# EUROPÄISCHE PATENTSCHRIFT

(11) **EP 2 900 301 B1**
(45) Veröffentlichungstag und Bekanntmachung des Hinweises auf die Patenterteilung: **14.09.2016**
(21) Anmeldenummer: 13776744.8
(22) Anmeldetag: 26.09.2013
(51) Int. Cl.: A61M 5/50, A61M 5/31, B65D 41/34

(54) **VORGEFÜLLTE SPRITZE**
PREFILLED SYRINGE
SERINGUE PRÉREMPLIE

(30) Priorität: 26.09.2012 US 201261706047 P
(43) Veröffentlichungstag der Anmeldung: 05.08.2015
(73) Patentinhaber: Transcoject GmbH, 24539 Neumünster (DE); Bayer Pharma Aktiengesellschaft, 13353 Berlin (DE)
(72) Erfinder: HEINZ, Jochen, 24220 Flintbek (DE)
(74) Vertreter: Patentanwälte Vollmann & Hemmer
(86) Internationale Anmeldenummer: PCT/EP2013/070140
(87) Internationale Veröffentlichungsnummer: WO 2014/049097

(56) Entgegenhaltungen:
- EP-A1- 1 410 817
- DE-A1- 10 247 965

## Beschreibung

Die Erfindung betrifft eine vorgefüllte Spritze, die einen Spritzenzylinder aufweist, der an einer Axialseite offen ausgebildet und mit einem Kolben verschlossen ist und der an der anderen Axialseite einen Luer-Lock-Anschluss aufweist, der an einer den Spritzenzylinder abschließenden Stirnwand angeformt und mit einem Stopfen verschlossen ist.

Derartige vorgefüllte Spritzen werden insbesondere in der Medizin vermehrt eingesetzt und sind mit einem fließfähigen Medium, d. h. typischerweise einem flüssigen oder pastösen Stoff, beispielsweise einem Medikament oder einem Kontrastmittel, gefüllt. Der Austrag dieses Mediums erfolgt mittels des Kolbens, der in den Spritzenzylinder eingeschoben wird, wodurch das Medium durch die Öffnung im Luer-Kegel des Luer-Lock-Anschlusses austritt. Um sicherzustellen, dass das im Spritzenzylinder befindliche Medium vor seiner Verwendung nicht aus dem LuerLock-Anschluss austritt und der Spritzenzylinder hermetisch abgeschlossen, insbesondere gegen das Eindringen von Keimen geschützt ist, ist ein Stopfen aus weichelastischem Material vorgesehen, welcher zumindest den Luer innerhalb des Luer-Lock-Anschlusses verschließt. Dabei ist insbesondere bei medizinischen Produkten sicherzustellen, dass von außen sofort erkennbar ist, wenn der Stopfen bereits einmal entfernt worden ist. Hierzu ist ein Originafitätsverschluss vorgesehen, welcher einen Trennsteg aufweist, der zerstört werden muss, um an den Stopfen zu gelangen, insbesondere den Stopfen vom Luer zu entfernen. Für aus Glas bestehende Spritzenzylinder, wie sie für medizinische Produkte nahezu ausschließlich Verwendung finden, zählt es zum Stand der Technik, einen aus Kunststoff bestehenden Originalitätsverschluss zusammen mit einem Luer-Anschluss sowie einem diesen verschließenden Stopfen am austragsseitigen Ende des Spritzenzylinders nach Art einer Rastverbindung aufzustecken (EP 0 397 951 A1). Problematisch bei derartigen Originalitätsverschlüssen ist es, dass diese einerseits so elastisch sein müssen, dass sie über einen Wulst am austragsseitigen Ende des Spritzenzylinders rastend aufgeschoben werden können, andererseits jedoch die Rastverbindung so ausgestaltet ist, dass der Originalitätsverschluss beim Öffnen an seiner Sollbruchstelle bricht und nicht die Rastverbindung gelöst wird. Dies ist fertigungstechnisch anspruchsvoll und erfordert eine hohe Genauigkeit bei der Herstellung.

Insoweit günstiger ist der aus DE 102 47 965 A1 bekannte Originalitätsverschluss, bei dem Spritzenzylinder und Luer-Lock einstückig als Kunststoffspritzgussteil ausgebildet sind und Stopfen und Originalitätsverschluss andererseits über eine stoffschlüssige Verbindung mit dem Spritzenzylinder verbunden sind. Nachteilig bei den dort beschriebenen Ausführungen ist, dass zunächst der Stopfen als gesondertes Bauteil hergestellt und in den ebenfalls als gesondertes Bauteil hergestellten Originalitätsverschluss eingefügt werden muss, wonach die so gebildete Einheit mit dem Spritzenzylinder verschweißt oder verklebt wird. Durch die im Originalitätsverschluss vorgesehene Sollbruchstelle verbleibt nach Öffnen des Originalitätsverschlusses stets ein Teil dieses Verschlusses spritzenseitig, was aus mehreren Gründen nachteilig ist. So ist zum einen der Luer an seinem Außenumfang nicht frei zugänglich, zum anderen birgt der zerstörte Trennsteg stets ein gewisses Verletzungsrisiko. Zwar ist dort alternativ vorgesehen, die Schweißnaht zwischen Originalitätsverschluss und Spritzenzylinder selbst als Trennsteg auszubilden, doch hat es sich als problematisch erwiesen, die Schweißnaht als Trennsteg auszubilden, auch verbleibt dort beim Zerstören des Trennstegs ein Verletzungsrisiko.

Schließlich hat sich die Verwendung von Kunststoff als Spritzenzylinder für vorgefüllte Spritzen in der Medizintechnik nicht bewährt, weshalb bei hochreinen Produkten stets aus Spezialglas gefertigte Spritzenzylinder Verwendung finden.

Vor diesem Hintergrund liegt der vorliegenden Erfindung die Aufgabe zugrunde, eine vorgefüllte Spritze zu schaffen, welche die oben erwähnten Nachteile weitgehend vermeidet, kostengünstig in der Herstellung und zuverlässig und sicher in der Anwendung ist.

Diese Aufgabe wird gemäß der Erfindung durch eine vorgefüllte Spritze mit den in Anspruch 1 angegebenen Merkmalen gelöst. Vorteilhafte Weiterbildungen der erfindungsgemäßen Spritze ergeben sich aus den Unteransprüchen, der nachfolgenden Beschreibung sowie der Zeichnung. Hierbei können gemäß der Erfindung die in den Unteransprüchen und der Beschreibung angegeben Merkmale jeweils für sich aber auch in geeigneter Kombination die erfindungsgemäße Lösung gemäß Anspruch 1 weiter ausgestalten.

Die erfindungsgemäße vorgefüllte Spritze weist einen Spritzenzylinder auf, der an einer Axialseite offen ausgebildet und mit einem Kolben verschlossen ist, wie dies bei derartigen Spritzen üblich ist, der jedoch an der anderen Axialseite einen Luer-Lock-Anschluss aufweist, der an einer den Spritzenzylinder abschließenden Stirnwand angeformt ist, d. h. der einstückig mit dem Spritzenzylinder und der Stirnwand als Kunststoffspritzgussteil ausgebildet ist. Das in der Spritze befindliche fließfähige Medium, welches pastös, flüssig oder gegebenenfalls auch gasförmig sein kann, ist durch den Spritzenzylinder umfänglich, durch den Kolben axial einerseits und durch die Stirnwand mit dem Luer-Lock-Anschluss und den weichelastischen Stopfen andererseits, eingeschlossen. Dabei schließt der Stopfen zumindest den Luer, d. h. den eigentlichen Austragskegel, innerhalb des Luer-Lock-Anschlusses dichtend ab. Der Stopfen ist nach außen hin von einem Originalitätsverschluss umgeben, der eine Sollbruchstelle aufweist. Der Originalitätsverschluss ist aus einem artgleichen oder artähnlichen Kunststoff wie der Spritzenzylinder ausgebildet und mit der Stirnwand des Spritzenzylinders verschweißt, wobei gemäß der Erfindung die Schweißverbindung die Sollbruchstelle bildet und der Stopfen in den Originalitätsverschluss eingegliedert ist. Da gemäß der Erfindung der Spritzenzylinder aus einem transparenten Kunststoff ausgebildet ist, kann der Inhalt der Spritze von außen eingesehen werden, und zwar sowohl durch den Spritzenzylinder als auch durch die Stirnwand bis in den Luer-Lock-Anschluss hinein. Vorzugsweise, aber nicht notwendigerweise, ist der Originalitätsverschluss ebenfalls aus einem transparenten Kunststoff gebildet. In jedem Falle ist er aus einem artgleichen oder artähnlichen Kunststoff gebildet, so dass die Schweißbarkeit zwischen dem Originalitätsverschluss und der Stirnwand des Spritzenzylinders gewährleistet ist.

Unter Stirnwand im Sinne der Erfindung ist der gesamte Bereich zwischen Luer-Lock-Anschluss und Außenumfang des Spritzenzylinders zu verstehen. Der Originalitätsverschluss kann also gegebenenfalls mit dem Spritzenzylinder fluchten oder diesen ganz oder teilweise radial überragen. Durch das Anschweißen des Originalitätsverschlusses an die Stirnwand des Spritzenzylinders ist der Schweißvorgang in einfacher Weise auch in Großserienfertigung durchzuführen und zu kontrollieren.

Dabei besteht der wesentliche Vorteil der erfindungsgemäßen Ausgestaltung darin, dass nicht wie beim Stand der Technik ein Trennsteg vorzusehen ist, sondern die Schweißverbindung selbst die Sollbruchstelle bildet, was insbesondere in Verbindung mit der transparenten Ausgestaltung der Bauteile den großen Vorteil bietet, dass nach Lösen des Originalitätsverschlusses von der Spritze, also nach Entfernen der Schweißverbindung bzw. Überwinden der Schweißverbindung, eine nahezu ansatzfreie Fläche an der Stirnwand des Spritzenzylinders verbleibt, die weder die Gefahr einer Verletzung birgt, noch die Sicht in das Innere des Spritzenzylinders einschränkt. Gerade letzteres ist im medizinischen Bereich von großer Bedeutung, um auszuschließen, dass versehentlich Gasblasen eingezogen werden oder auch nur um den kontrollierten Ausfluss des Mediums vom Spritzenzylinder in den Luer-Anschluss durch Sichtkontrolle überprüfen zu können.

Insbesondere wenn der Originalitätsverschluss, was vorteilhaft ist, ebenfalls transparent ausgebildet ist, kann bereits vor Öffnen desselben von außen der korrekte Sitz des Stopfens und die Unversehrtheit des innerhalb des Spritzenzylinders befindlichen Mediums festgestellt werden. Die erfindungsgemäße Lösung stellt somit im Vergleich zum Stand der Technik eine wesentlich kostengünstigere Lösung als die bisher aus Glas bestehenden Spritzenzylinder dar, zugleich jedoch eine wesentlich sichere und besser zu handhabende Lösung als dies bei bisherigen aus Kunststoff bestehenden Spritzen bekannt ist.

Besonders vorteilhaft ist es, wenn der Stopfen und der Originalitätsverschluss im 2K-Spritzgussverfahren hergestellt sind. Hierdurch ist es möglich, den Stopfen aus einem geeigneten weichelastischen, thermoplastischen Material zu bilden und den Originalitätsverschluss aus einem vergleichsweise harten Kunststoff, ähnlich dem des Spritzenzylinders. 2K-Spritzgussverfahren sind technisch gut beherrschbar und stellen somit eine kostengünstige Möglichkeit dar, zwei an sich voneinander unabhängige Bauteile in einem Spritzgussverfahren quasi einstückig herzustellen. Somit entfällt die Montage des Stopfens innerhalb des Originalitätsverschlusses, wodurch die Fertigungskosten der vorgefüllten Spritze weiter gesenkt werden.können.

Gemäß einer vorteilhaften Weiterbildung der Erfindung sind der Originalitätsverschluss und der Stopfen formschlüssig miteinander verbunden. Ein solcher Formschlussverbund stellt sicher, dass der Stopfen formschlüssig innerhalb des Öriginalitätsverschlusses angeordnet ist, also stets mit diesem zusammen gehandhabt wird. Eine solche Formgestaltung der Bauelemente bietet den Vorteil, dass die im 2K-Spritzgussverfahren verwendeten Werkstoffe für Stopfen und Originalitätsverschluss mit größerem Freiheitsgrad gewählt werden können, da diese weder eine stoffschlüssige, noch eine sonstige Haftverbindung eingehen müssen, sondern im Extremfall ausschließlich durch den Formschluss miteinander verbunden sein können. Dabei ist der Formschluss vorteilhaft zwischen Stopfen und Originalitätsverschluss nach außen und in Drehrichtung um die Mittellängsachse gebildet. Durch diese Anordnung ist sichergestellt, dass sowohl beim Drehen als auch beim Kippen des Originalitätsverschlusses an der Spritze der Stopfen stets mitbewegt wird und damit beim Zerstören der Sollbruchstelle, d. h. der Schweißverbindung zwischen Originalitätsverschluss und der Stirnwand des Spritzenzylinders, zuverlässig auch der Stopfen mitbewegt und schließlich entfernt wird. Da der Originalitätsverschluss den Stopfen umfänglich umgibt, kann über diesen bei entsprechender Formschlussverbindung auf den Stopfen eine vergleichsweise große Handkraft aufgebracht werden, so dass auch bei festem Sitz des Stopfens innerhalb des Luer-Anschlusses bzw. des Luer-Lock-Anschlusses, wenn der Stopfen den Luer-Anschluss auch umfänglich umgibt, dennoch ein einfaches Lösen mit Handkraft gewährleistet ist.

Vorteilhaft besteht der Spritzenzylinder mit dem angeformten Luer-Lock-Anschluss und gegebenenfalls auch der Originalitätsverschluss aus einem Polyolefin. Aus dieser Gruppe der Kunststoffe sind zahlreiche Verbindungen bekannt, welche die speziellen Anforderungen an den Werkstoff für eine vorgefüllte Spritze erfüllen, d. h. insbesondere transparent ausgebildet sind, eine hohe Maßhaltigkeit beim Spritzgießen gewährleisten und eine hohe Barriere gegen Durchdringen des in dem Spritzenzylinder gelagerten Mediums bieten. Ein kostengünstiger Kunststoff für den Spritzenzylinder und den angeformten Luer-Lock-Anschluss bzw. den Originalitätsverschluss ist Polypropylen. Alternativ kann der Spritzenzylinder vorteilhaft auch aus einem Cyclo-Olefin-Copolymer bestehen oder aus anderen geeigneten thermoplastischen Werkstoffen.

Der Spritzenzylinder besteht zweckmäßigerweise aus einem Barrierekunststoff, wobei die Barriereeigenschaften auf das Füllmedium abgestimmt sind.

Vorteilhaft besteht der Stopfen aus einem thermoplastischen Polymer und ist im Vergleich zum Material des Spritzenzylinders eher weich und elastisch. Ein besonders geeigneter Werkstoff hierfür ist ein thermoplastisches Polyurethan, welches in der Spritzgießtechnik, insbesondere der 2K-Spritzgusstechnik, gut handhabbar ist.

Die Erfindung ist nachfolgend anhand eines in der Zeichnung dargestellten Ausführungsbeispiels näher erläutert. Es zeigen:
- Fig. 1: in stark vereinfachter, schematischer Darstellung das austragsseitige Ende einer vorgefüllten Spritze mit aufgesetztem Originalitätsverschluss im Längsschnitt und
- Fig. 2: einen Schnitt längs der Schnittlinie A-A in Fig. 1.

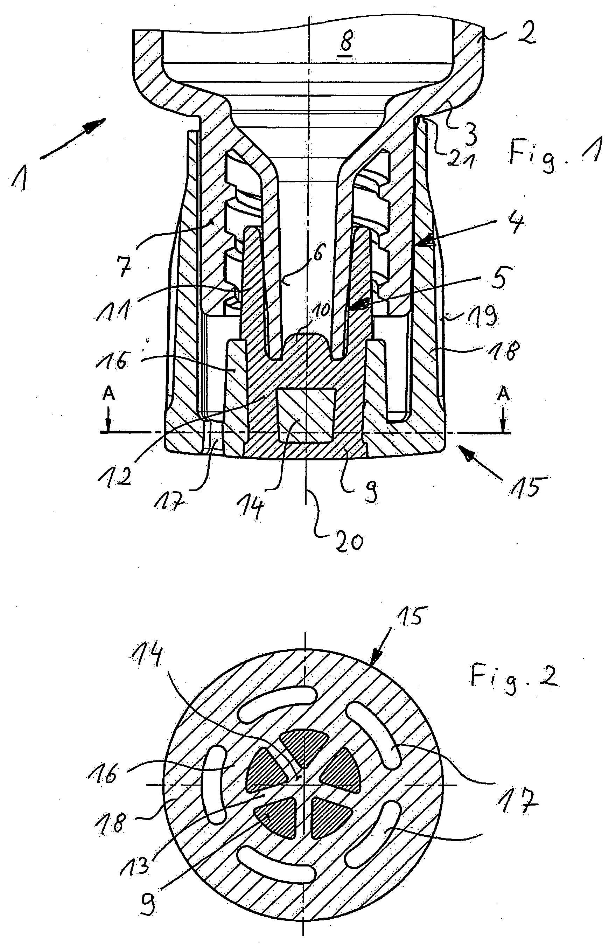

In Fig. 1 ist nur der austragsseitige Teil einer vorgefüllten Spritze 1 dargestellt. Diese Spritze weist einen langgestreckten Spritzenzylinder 2 auf, der an seinem in der Fig. 1 nicht sichtbaren axialen Ende offen ausgebildet und durch einen verschiebbar innerhalb des Zylinders 2 geführten, ebenfalls nicht dargestellten Stopfen verschlossen ist. An der anderen in Fig. 1 dargestellten Axialseite ist der Spritzenzylinder 2 durch eine Stirnwand 3 abgeschlossen, an welcher ein Luer-Lock-Anschluss 4 angeformt ist. Spritzenzylinder 2, Stirnwand 3 und Luer-Lock-Anschluss 4 sind als einstückiges Kunststoffspritzgussteil ausgebildet und bestehen aus einem Werkstoff, wie er einleitend beispielhaft und vorteilhaft angegeben ist. Der Luer-Lock-Anschluss 4 besteht in an sich bekannter Weise aus einem inneren Luer-Kegel 5, welcher einem üblichen Luer-Anschluss entspricht und der einen inneren Durchgangskanal 6 zum Spritzenzylinder 2 aufweist. Umgeben wird der Luer-Anschluss 5 von einem zylindrischen Abschnitt 7, der an seiner Innenseite ein Innengewinde trägt und zusammen mit dem Luer-Anschluss 5 den Luer-Lock-Anschluss 4 in an sich bekannter Weise bildet.

Das im Spritzenzylinder 2 befindliche Medium 8 ist durch den Spritzenzylinder 2 umfänglich, an der offenen, in Fig. 1 nicht sichtbaren Axialseite durch den Stopfen und an der durch die Stirnwand 3 und den Luer-Lock-Anschluss 4 abgeschlossenen anderen Axialseite mittels eines Stopfens 9 hermetisch abgeschlossen. Der Stopfen 9 besteht aus einem weichelastischen thermoplastischen Werkstoff und weist einen kegelförmig in den Durchgangskanal 6 des Luer-Anschlusses 5 gerichteten Vorsprung 10 auf, der den Durchgangskanal 6 nach außen hin dicht verschließt. An den Vorsprung 10 schließt sich ein ringförmiger Abschnitt 11 mit radialem Abstand an, der den Vorsprung 10 zum Spritzenzylinder 2 hin deutlich in seiner axialen Länge überragt. Dieser ringförmige Abschnitt 11 umgibt den Luer-Kegel 5 umfänglich, dabei ist zwischen dem Vorsprung 10 und dem ringförmigen Abschnitt 11 eine ringförmige Nut gebildet, in welche das freie Ende des Luer-Kegels 5 eingreift. Die Abdichtung erfolgt einmal durch den Vorsprung 11, welcher das freie Ende des Durchgangskanals 6 dicht verschließt, und zum anderen durch den ringförmigen Abschnitt 11, der sich dichtend an den Außenumfang des Luer-Kegels 5 anlegt. Der Vorsprung 10 und der ringförmige Abschnitt 11 gehen in einen im Wesentlichen zylindrischen Abschnitt 12 über, der durch fünf radial verlaufende Stege 13 durchsetzt ist, die in einem zentralen Raum 14 münden.

Im Bereich der Stege 13 und des zentralen Raums 14 ist der Stopfen 9 durch ein einen Originalitätsverschluss 15 bildendendes Kunststoffbauteil durchsetzt. Dieser Originalitätsverschluss 15 weist neben den in die Freiräume 13 und 14 ragenden Abschnitten einen ringförmigen Abschnitt 16 auf, der den Stopfen 9 bis nahe zum axialen Ende des Vorsprungs 10 umgibt. Dieser ringförmige Abschnitt 16 ist über einen durch ringförmig angeordnete Ausnehmungen 17 unterbrochenen Bereich mit einem radial aufgeweiteten, ringförmigen Abschnitt 18 verbunden, welcher mit seinem zylindrischen Innendurchmesser mit geringem Spiel am Außenumfang des zylindrischen Abschnitts 7 des Luer-Lock-Anschlusses 4 anliegt und bis zur Stirnwand 3 reicht, wo er abschnittsweise mit dieser verschweißt ist. Dabei läuft der ringförmige Abschnitt 18 zur Stirnwand 3 verjüngend zu, weist achsparallele Rippen 19 auf, welche die Griffigkeit dieses Abschnitts erhöhen.

Wie aus Fig. 1 ersichtlich ist, ist der ringförmige Abschnitt 18 nicht voll umfänglich mit der Stirnwand 3 verschweißt, sondern nur in Abschnitten, ähnlich wie die Anordnung der Ausnehmungen 17 auf der anderen Axialseite des Originalitätsverschlusses. Dadurch, dass der ringförmige Abschnitt 18 nur abschnittsweise mit der Stirnwand 3 des Spritzenzylinders 2 verschweißt ist, wird eine Sollbruchstelle zwischen diesen beiden Bauteilen gebildet, die durch einfache Handkraft aufgebrochen werden kann. Dabei wird die Schweißverbindung gelöst, d. h. nach Öffnen des Originalitätsverschlusses verbleibt die Stirnwand 3 nahezu in ihrer ursprünglichen Form, d. h. sie bleibt eben und glatt sowie im Wesentlichen durchsichtig, so dass der Einblick in das Innere der Spritze 1 und somit zum Befüllmedium 8 durch den transparenten Kunststoff des Spritzenzylinders möglich ist. Es ist also insbesondere der Austragsbereich der Spritze 1 nach Entfernen des Originalitätsverschlusses 15 einsehbar und damit in einfacher Weise optisch kontrollierbar. Auch ist die Oberfläche der Stirnwand 3 glatt und im Wesentlichen frei von Resten des Originalitätsverschlusses 15.

Der Stopfen 9, der in der dargestellten Ausführungsförm nicht transparent ausgebildet ist, ist mit dem Originalitätsverschluss 15, der ebenfalls aus einem transparenten thermoplastischen Kunststoff besteht, durchsetzt, so dass zum einen nach der Montage dieses aus Stopfen 9 und Originalitätsverschluss 15 bestehenden, im 2K-Spritzgussverfahren hergestellten Bauteils stets der korrekte Sitz des Stopfens 9 einerseits sichergestellt ist und andererseits beim Aufbrechen der Schweißverbindung auch ein Bewegen und damit Entfernen des Stopfens 9 gewährleistet ist. Es ist ersichtlich, dass die den Stopfen 9 und den Originalitätsverschluss 15 bildenden Werkstoffe keinerlei stoffschlüssigen Verbund miteinander eingehen müssen, sondern dass der konstruktionsbedingte Formschluss zwischen diesen Bauteilen ausreicht, diese fest und unlösbar miteinander zu verbinden.

Wie die Schnittdarstellung gemäß Fig. 1 verdeutlicht, ist das im 2K-Spritzgiessverfahren aus Stopfen 9 und Originalitätsverschluss 15 gebildete Bauteil konstruktiv so ausgestaltet, dass beim Aufsetzen dieses Bauteils auf die Spritze die Innenseite des ringförmigen Abschnitts 18 auf der Außenseite des zylindrischen Abschnitts 7 des Luer-Lock-Anschlusses 4 geführt wird. Durch diese zylindrische Führung wird zunächst der ringförmige Abschnitt 18 auf den Luer-Anschluss 5 aufgesetzt, wobei in der Endphase des Montagevorgangs der Vorsprung 10 in das Ende des Durchgangskanals 6 dichtend eingegliedert wird. Durch diese Führung ist sichergestellt, dass der Stopfen 9 stets bestimmungsgemäß den Luer-Anschluss 5 verschließt. Durch die Anordnung der Ringabschnitte 21, mit denen der Originaütätsverschluss 15 mit der Stirnwand 3 der Spritze verschweißt ist, können diese Schweißnahtabschnitte auch in Großserienfertigung in einfacher Weise hergestellt werden, da die Bereiche gut zugänglich sind. Auch kann dieser Fertigungsprozess nicht nur durch Sichtkontrolle, sondern auch durch geeignete andere Prüfverfahren überwacht werden. Schließlich ist an dieser exponierten Stelle der Spritze sofort erkennbar, wenn der Originalitätsverschluss 15 aufgebrochen ist, d. h. die Schweißverbindung von der Stirnwand 3 gelöst ist. Dies wird insbesondere dadurch gut sichtbar, dass die Schweißung nicht durchgängig umlaufend, sondern nur im Bereich der stirnseitigen Abschnitte 21 erfolgt, so dass beim Entfernen und Wiederaufsetzen des Originalitätsverschlusses 15 typischerweise eine Drehung um die Mittellängsachse 20 erfolgt, welche an der Stirnwand 3 sichtbar ist, da dann die Schweißstellen nicht mehr mit den stirnseitigen Abschnitten 21 fluchten.

### Bezugszeichenliste

- 1: - Spritze
- 2: - Spritzenzylinder
- 3: - Stirnwand
- 4: - Luer-Lock-Anschluss
- 5: - Luer-Kegel, Luer-Anschluss
- 6: - Durchgangskanal
- 7: - zylindrischer Abschnitt
- 8: - Befüllmedium
- 9: - Stopfen
- 10: - Vorsprung
- 11: - ringförmiger Abschnitt
- 12: - zylindrischer Abschnitt
- 13: - Stege
- 14: - zentraler Raum
- 15: - Originalitätsverschluss
- 16: - ringförmiger Abschnitt
- 17: - Ausnehmungen
- 18: - ringförmiger Abschnitt
- 19: - Rippen
- 20: - Mittellängsachse
- 21: - stirnseitige Abschnitte

## Patentansprüche

1. Vorgefüllte Spritze mit einem Spritzenzylinder (2), der an einer Axialseite offen ausgebildet und mit einem Kolben verschlossen ist, der an der anderen Axialseite einen Luer-Lock-Anschluss, (4) aufweist, der an einer den Spritzenzylinder (2) abschließenden Stirnwand (3) angeformt ist, mit einem in den Spritzenzylinder (2) eingefüllten fließfähigen Medium (8), mit einem zumindest den Luer (5) verschließenden weichelastischen Stopfen (9) und mit einem den Stopfen (9) nach außen hin umgebenden Originalitätsverschluss (15) mit einer Sollbruchstelle, wobei der Spritzenzylinder (2), die Stirnwand (3) und der Luer-Lock-Anschluss (4) einstückig als transparentes Kunststoffspritzgussteil ausgebildet sind, der Originalitätsverschluss (15) aus einem artgleichen oder artähnlichen Kunststoff wie der Spritzenzylinder (2) besteht und mit der Stirnwand (3) des Spritzenzylinders (2) verschweißt ist, wobei die Schweißverbindung die Sollbruchstelle bildet und der Stopfen (9) in den Originalltätsverschluss (15) eingegliedert ist.

2. Vorgefülite Spritze nach Anspruch 1, bei der Stopfen (9) und der Originalitätsverschluss (15) im 2K-Spritzgussverfahren hergestellt sind.

3. Vorgefüllte Spritze nach Anspruch 1 oder 2, bei der der Originalitätsverschluss (15) und der Stopfen (9) formschlüssig miteinander verbunden sind.

4. Vorgefüllte Spritze nach einem der vorhergehenden Ansprüche, **dadurch gekennzeichnet, dass** der Originalitätsverschluss (15) transparent ausgebildet ist.

5. Vorgefüllte Spritze nach einem der vorhergehenden Ansprüche, bei der der Formschluss zwischen Stopfen (9) und Originalitätsverschluss (15) nach Außen und in Drehrichtung um die Mittellängsachse (20) gebildet ist.

6. Vorgefüllte Spritze nach einem der vorhergehenden Ansprüche, bei der der Spritzenzylinder (2) aus einem Polyolefin besteht.

7. Vorgefüllte Spritze nach Anspruch 5, bei der der Spritzenzylinder (2) aus Polypropylen besteht.

8. Vorgefüllte Spritze nach einem der vorhergehenden Ansprüche, bei der der Spritzenzylinder (2) aus einem Cyclo-Olefin-Copölymer besteht.

9. Vorgefüllte Spritze nach einem der vorhergehenden Ansprüche, bei der der Spritzenzylinder (2) aus einem Barrierekunststoff besteht, dessen Barriereeigenschaften auf das Füllmedium (8) abgestimmt sind.

10. Vorgefüllte Spritze nach einem der vorhergehenden Ansprüche, bei der der Stopfen (9) aus einem thermoplastischen Polymer (TPE) besteht.

11. Vorgefüllte Spritze nach einem der vorhergehenden Ansprüche, bei der der Stopfen (9) aus einem thermoplastischen Polyurethan (TPU) besteht.

## Claims

1. A prefilled syringe with a syringe cylinder (2) which at one axial side is designed in an open manner and is closed by a plunger, and which comprises a Luer lock connection (4) at the other axial side, said Luer lock connection (4) being integrally formed on a face wall (3) closing off the syringe cylinder (2), with a flowable medium (8) filled into the syringe cylinder (2), with a soft-elastic plug (9) which closes at least the Luer (5) and with tamper-evident closure (15) which surrounds to the plug (9) to the outside and is with a predetermined breakage location, wherein the syringe cylinder (2), the face wall (3) and the Luer lock connection (4) are designed as one piece as a transparent plastic injection moulded part, the tamper-evident closure (15) consists of the same type or similar type of plastic as the syringe cylinder (2) and is welded to the face wall (3) of the syringe cylinder (2), wherein the weld connection forms the predetermined breakage location and the plug (9) is integrated into the tamper-evident closure (15).

2. A prefilled syringe according to claim 1, with which the plug (9) and the tamper-evident closure (15) are manufactured with the 2K injection moulding method.

3. A prefilled syringe according to claim 1 or 2, with which the tamper-evident closure (15) and the plug (9) are positively connected to one another.

4. A prefilled syringe according to one of the preceding claims, **characterised in that**, the tamper-evident closure (15) is designed in a transparent manner.

5. A prefilled syringe according to one of the preceding claims, with which the positive-fit between the plug (9) and the tamper-evident closure (15) is formed to the outside and about the middle longitudinal axis (20) in the rotation direction.

6. A prefilled syringe according to one of the preceding claims, with which the syringe cylinder (2) consist of polyolefin.

7. A prefilled syringe according to claim 5, with which the syringe cylinder (2) consist of polypropylene.

8. A prefilled syringe according to one of the preceding claims, with which the syringe cylinder (2) consists of a cyclo-olefin copolymer.

9. A prefilled syringe according to one of the preceding claims, with which the syringe cylinder (2) consist of a barrier plastic, whose barrier characteristics are matched to the filling medium (8).

10. A prefilled syringe according to one of the preceding claims, with which the plug (9) consists of a thermoplastic polymer (TPE).

11. A prefilled syringe according to one of the preceding claims, with which the plug (9) consist of a thermoplastic polyurethane (TPU).

## Revendications

1. Seringue préremplie comportant un cylindre de seringue (2), qui est conçu ouvert sur un côté axial et est obturé par un piston, qui présente sur l'autre côté axial un embout Luer-Lock (4), qui est rapporté au niveau d'une paroi frontale (3) bouchant le cylindre de seringue (2), comprenant un milieu fluide (8) introduit dans le cylindre de seringue (2), comportant un bouchon (9) souple obturant au moins le Luer (5), et une obturation inviolable (15) qui entoure le bouchon (9) vers l'extérieur et possède un point de rupture, le cylindre de seringue (2), la paroi frontale (3) et l'embout Luer-Lock (4) étant conçus en une seule pièce sous la forme d'une pièce plastique transparente moulée par injection, l'obturation inviolable (15) étant constituée d'un matériau plastique de même type ou d'un type analogue à celui du cylindre de seringue (2) et étant soudée à la paroi frontale (3) du cylindre de seringue (2), l'assemblage soudé formant le point de rupture, et le bouchon (9) étant intégrés dans l'obturation inviolable (15).

2. Seringue préremplie selon la revendication 1, dans laquelle le bouchon (9) et l'obturation inviolable (15) sont fabriqués par un procédé de moulage par injection de deux composants.

3. Seringue préremplie selon la revendication 1 ou 2, dans laquelle l'obturation inviolable (15) et le bouchon (9) sont reliés l'un à l'autre par une liaison par correspondance de forme.

4. Seringue préremplie selon l'une des revendications précédentes, **caractérisée en ce que** l'obturation inviolable (15) est conçue transparente.

5. Seringue préremplie selon l'une des revendications précédentes, dans laquelle la liaison par correspondance de forme entre le bouchon (9) et l'obturation inviolable (15) est formée vers l'extérieur, et dans le sens de rotation autour de l'axe longitudinal central (20).

6. Seringue préremplie selon l'une des revendications précédentes, dans laquelle le cylindre de seringue (2) est constitué d'une polyoléfine.

7. Seringue préremplie selon la revendication 5, dans laquelle le cylindre de seringue (2) est constitué de polypropylène.

8. Seringue préremplie selon l'une des revendications précédentes, dans laquelle le cylindre de seringue (2) est constitué d'un copolymère de cyclo-oléfine.

9. Seringue préremplie selon l'une des revendications précédentes, dans laquelle le cylindre de seringue (2) est constitué d'un plastique barrière dont les propriétés barrière sont adaptées au milieu de remplissage (8).

10. Seringue préremplie selon l'une des revendications précédentes, dans laquelle le bouchon (9) est constitué d'un polymère thermoplastique (TPE).

11. Seringue préremplie selon l'une des revendications précédentes, dans laquelle le bouchon (9) est constitué d'un polyuréthanne thermoplastique (TPU).
